# FASCICULE DE BREVET EUROPEEN

(11) **EP 1 136 124 B1**
(45) Date de publication et mention de la délivrance du brevet: **04.05.2005**
(21) Numéro de dépôt: 01400511.0
(22) Date de dépôt: 28.02.2001
(51) Int. Cl.: B01J 31/22, B01J 31/14, C08F 10/08, C07F 15/04

(54) **Composition catalytique et procédé de dimérisation, de codimérisation et d'oligomérisation des oléfines**
Katalytische Zusammenstellung und Verfahren für die Dimerisierung, Codimerisierung und Oligomerisierung von Olefinen
Catalytic composition and process of dimerisation, codimerisation and oligomerisation of olefins

(30) Priorité: 23.03.2000 FR 0003820
(43) Date de publication de la demande: 26.09.2001
(73) Titulaire: Institut Français du Pétrole, 92852 Rueil-Malmaison Cedex (FR)
(72) Inventeur: Olivier-Bourbigou, Hélène, 92500 Rueil-Malmaison (FR); Commereuc, Dominique, 92190 Meudon (FR); Harry, Stéphane, 78360 Montesson (FR)

(56) Documents cités:
- EP-A- 0 798 041
- DE-A- 4 447 066
- US-A- 5 166 114
- US-A- 5 324 799

## Description

La présente invention concerne la dimérisation, la codimérisation et l'oligomérisation catalytique des oléfines.

Elle a pour objet une formule catalytique résultant de la dissolution d'un complexe du nickel contenant au moins un ligand carbène hétérocyclique avec au moins un halogénure d'hydrocarbylaluminium et éventuellement au moins un solvant organique. La présente invention a également pour objet l'utilisation de cette composition catalytique dans les procédés de dimérisation, de codimérisation et/ou d'oligomérisation des oléfines.

Il est connu de préparer des catalyseurs de dimérisation ou de codimérisation de monooléfines telles que l'éthylène, le propylène, les butènes ou les pentènes. Parmi ces catalyseurs, on peut citer notamment à titre d'exemples : les produits d'interaction des halogénures de π-allyl nickel. phosphine avec les acides de Lewis (brevet français FR-B-1 410 430), les produits d'interaction des halogénures de nickel phosphine avec les acides de Lewis (brevet US-A-3 485 881) et les produits d'interaction de certains carboxylates de nickel avec les halogénures d'hydrocarbylaluminium (brevet US-A-3 321 546).

Presque tous ces catalyseurs mettent en oeuvre un ligand tel qu'un composé organique du phosphore. Or il est préférable de pouvoir disposer de catalyseurs d'oligomérisation sans phosphore. On pourrait utiliser des catalyseurs dans lesquels le nickel se trouve déposé sur un support minéral comportant des sites acides, comme la silice, l'alumine ou les silice-alumines. Cependant, il s'agit là de catalyseurs solides, contrairement aux catalyseurs en phase liquide de l'invention.

Certains complexes organométalliques du nickel contenant des ligands carbènes hétérocycliques ont été décrits dans l'art antérieur (demande internationale WO-A-99/06 004, brevet US-A-5 728 839 et demande de brevet EP-A-0 798 041). De tels complexes ont l'avantage d'être très stables. Plus particulièrement, ces ligands monocarbènes ou bicarbènes conduisent à des complexes du nickel stables thermiquement et chimiquement surtout vis-à-vis de l'oxydation. Ces ligands carbènes ont fait l'objet d'une revue dans Angew. Chem. Int. Ed. Engl. 1997, 36, 2162. Ce sont des ligands σ-donneurs et π-accepteurs qui forment avec les métaux de transition des liaisons très stables. Leurs propriétés électroniques peuvent être comparées à celles des trialkylphosphines basiques.

On peut encore citer le brevet US-A-5 166 114, qui décrit une composition catalytique pour la polymérisation de l'éthylène contenant un composé organique du Ni(II), un organoaluminium, une phosphjine organique et un polymère perflurosulfonate et le brevet US-A-5 324 799 qui décrit une composition catalytique pour la polymérisation de l'éthylène contenant un complexe organique du Ni(II), un organoaluminium et uri amino-bis(imino)-phosphorane.

Il a maintenant été trouvé que la mise en contact
- d'un complexe du nickel portant au moins un ligand monocarbène ou bicarbène répondant par exemple aux formules (I) et (II) données ci-après ;
- avec au moins un halogénure d'hydrocarbylaluminium ;
- et éventuellement un solvant organique
conduisait à un système actif pour la dimérisation, la codimérisation et/ou l'oligomérisation des oléfines.

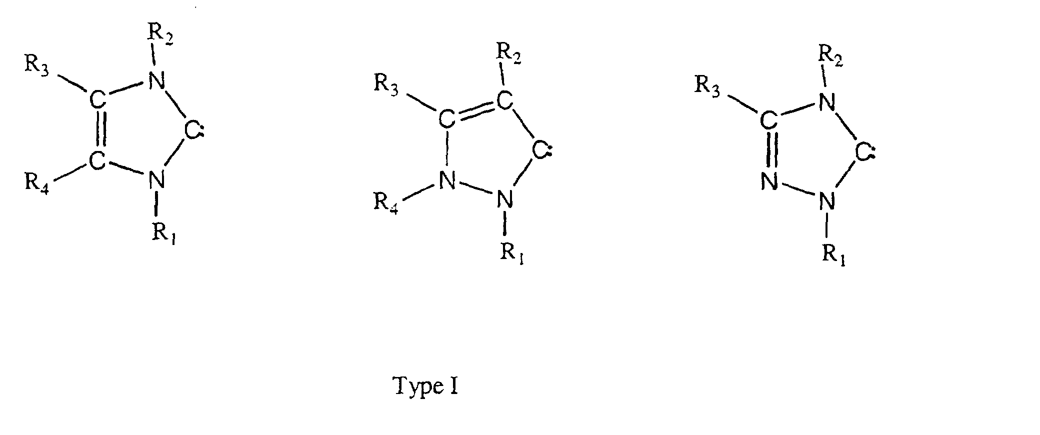

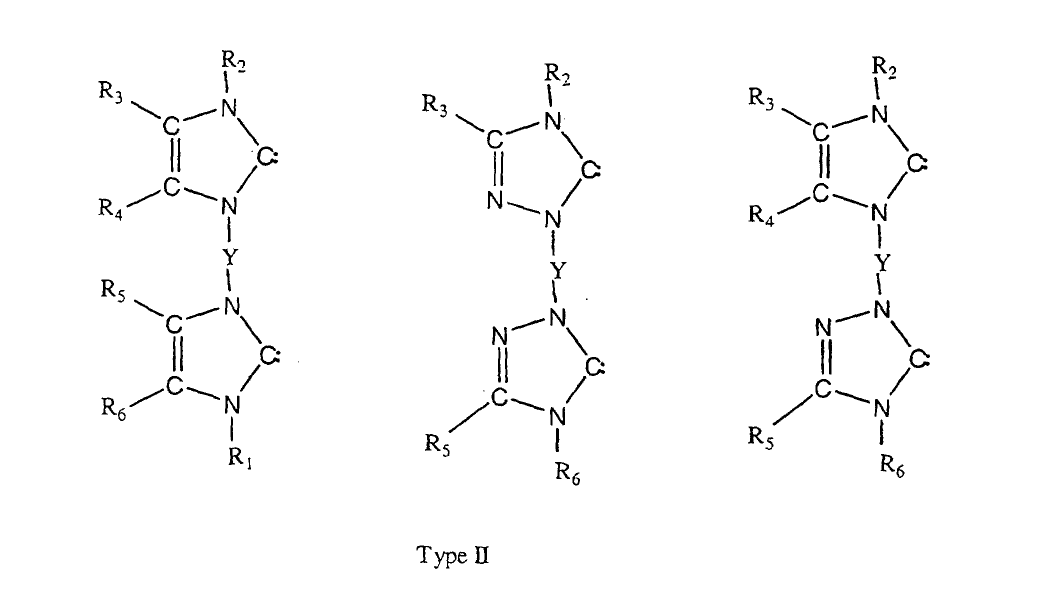

Les composés du nickel utilisés selon l'invention sont des sels de nickel ou des composés organométalliques, chargés ou non, qui répondent à la formule générale (déjà décrite dans la demande de brevet EP-A-0 798 041) :

(NiₐX_{b}Y_{d}L_{c})ⁿ (A)ₙ

dans laquelle :
- a, b, c, d et n sont des nombres entiers avec a égal à 1, 2 ou 3 ; b de 0 à 2 fois a ; d de 0 à 2 fois a ; c de 1 à 4 fois b ; n égal à 0, 1 ou 2 ;
- X et Y, identiques ou différents, représentent chacun un ligand mono- ou multi-chélatant, chargé ou non ; à titre d'exemples, on pourra citer les halogénures, les carboxylates (par exemple l'éthyl-2-hexanoate), l'acétylacétonate, le sulfate, les phénates, les mono- et di-oléfines, les π-aromatiques, les radicaux alkyles ou aryles, les phosphines, les phosphites et le monoxyde de carbone ;
- L est un mono- ou bi-carbène hétérocyclique qui répond par exemple à l'une des formules générales (I) et (II) ci-dessus, dans lesquelles R₁, R₂, R₃, R₄, R₅ et R₆, identiques ou différents, représentent chacun l'hydrogène, un groupe hydrocarboné, aliphatique, saturé ou insaturé, ou aromatique, comprenant de 1 à 12 atomes de carbone, et Y représente un reste bivalent aliphatique de 1 à 4 atomes de carbone ; et
- A est un anion faiblement coordinant ; à titre d'exemples, on pourra citer les anions tétrafluoroborate, hexafluorophosphate, tétraphénylborate et leurs dérivés, les anions tétrachloroaluminate, hexafluoroantimonate, trifluoroacétate, trifluorométhylsulfonate et acétate.

Les carbènes hétérocycliques L peuvent être générés à partir des sels d'imidazolium ou bis(azolium) correspondants, par déprotonation. Le métal de transition peut jouer le rôle de réducteur.

A titre d'exemples non limitatifs de ligands mono- ou bicarbènes hétérocycliques, on citera les ligands carbènes décrits par les formules (1), (2) et (3) données ci-après.

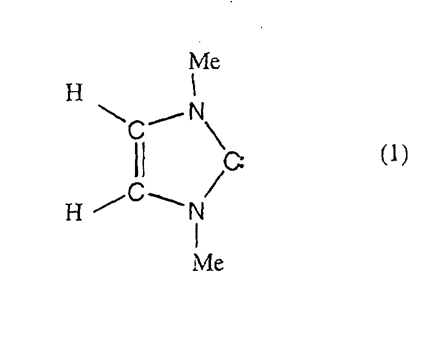

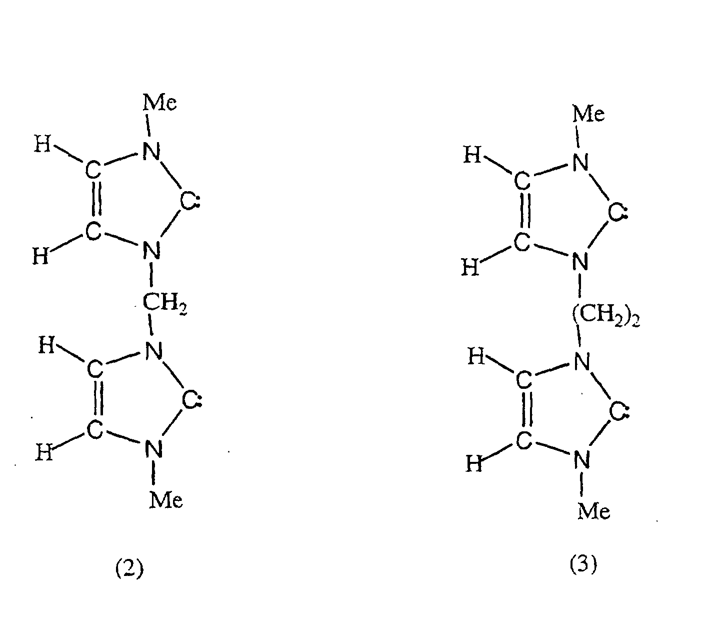

A titre d'exemples non limitatifs de composés du nickel utilisables selon l'invention, on peut citer les complexes NiCl₂, [diméthyl-1,3-imidazolylidène-2]₂ ; Nil₂,[diméthyl-1,3-imidazolylidène-2]₂ ; le chlorure de π-allyl nickel (diméthyl-1,3-imidazolylidène-2) ; Nil₂,[diméthyl-1,1'-imidazol-diylidène-2,2'-méthylène-3,3']₂ et Nil₂,[diméthyl-1,1'-imidazol-diylidène-2,2'-éthylène-3,3']₂.

Les dérivés halogénures d'hydrocarbylaluminium utilisés selon l'invention ont pour formule générale AlRₓX₃₋ₓ, dans laquelle R est un reste hydrocarboné comportant de 1 à 12 atomes de carbone, qui peut être alkyle, linéaire ou ramifié, cycloalkyle, aryle ou aralkyle, X étant le chlore ou le brome et x est un nombre de 1 à 3. A titre d'exemples non limitatifs de ces dérivés, on peut citer le sesquichlorure d'isobutylaluminium, le sesquichlorure d'éthylaluminium, le dichloroisobutylaluminium, le dichloroéthylaluminium et le chlorodiéthylaluminium.

Les constituants de la formule catalytique peuvent être mélangés dans un ordre quelconque.

Le solvant facultatif permettant d'effectuer le mélange des constituants de la formule catalytique et dans lequel peut être effectuée la catalyse est un solvant hydrocarboné, par exemple un alcane ou un hydrocarbure aromatique, ou encore un hydrocarbure halogéné ou encore le mélange d'oléfines produit dans la réaction d'oligomérisation.

Le rapport molaire du composé organique de l'aluminium au composé du nickel, exprimé par le rapport Al/Ni, est par exemple de 2/1 à 50/1 et de préférence de 2/1 à 20/1.

Les oléfines susceptibles d'être dimérisées, codimérisées ou oligomérisées par les compositions catalytiques selon l'invention sont l'éthylène, le propylène, les n-butènes et les n-pentènes, seuls ou en mélange, purs ou dilués par un ou plusieurs alcane(s), tels qu'on les trouve dans des « coupes » issues des procédés de raffinage du pétrole, comme le craquage catalytique ou le craquage à la vapeur.

La réaction catalytique de dimérisation, d'oligomérisation ou de codimérisation des oléfines qui est aussi un objet de l'invention, peut être conduite en système fermé, en système semi-ouvert ou en continu avec un ou plusieurs étages de réaction. La température de réaction peut être de -40 à +80 °C, de préférence de -20 à +50 °C, dans des conditions de pression telles que les réactifs soient maintenus au moins en majorité en phase liquide ou en phase condensée. La chaleur engendrée par la réaction peut être éliminée par tous les moyens connus de l'homme du métier.

Le procédé peut être mis en oeuvre dans un réacteur à un ou plusieurs étages de réaction en série, la charge oléfinique et/ou la composition catalytique au préalable pré-conditionnée étant introduites en continu, soit dans le premier étage, soit dans le premier et un autre quelconque des étages. A la sortie du réacteur, le catalyseur peut être désactivé, par exemple par injection d'ammoniac et/ou d'une solution aqueuse de soude et/ou d'une solution aqueuse d'acide sulfurique. Les oléfines non converties et les alcanes éventuellement présents dans la charge sont ensuite séparés des oligomères par distillation.

Les produits du présent procédé peuvent trouver une application par exemple comme composants de carburants pour automobiles et/ou comme charges dans un procédé d'hydroformylation pour la synthèse d'aldéhydes et d'alcools.

Les exemples suivants illustrent l'invention sans en limiter la portée.

### EXEMPLE 1 : Préparation: du complexe de nickel Nil₂,[diméthyl-1,3-imidazolylidène-2]₂ :

Le complexe de nickel est préparé par une amélioration de la synthèse décrite dans Organometallics, 1997, 16, 2209.

Dans un tube de type Schlenk, on agite fortement l'acétate de nickel préalablement séché (6 mmoles) avec l'iodure de diméthyl-1,3-imidazolium (12 mmoles) dans le nitrométhane (60 mL). On chauffe à 150 °C en tirant sous le vide de la pompe pendant une heure. On laisse refroidir, puis on extrait le complexe rouge formé avec du tétrahydrofuranne chaud (500 mL). Le THF est ensuite évaporé sous vide et le composé rouge est lavé à l'éther diéthylique (140 mL). Un deuxième lavage avec de l'éthanol absolu (15 mL) est nécessaire pour éliminer l'iodure de diméthyl-1,3-imidazolium qui n'a pas réagi.

### EXEMPLE 2 : Dimérisation du butène

Un réacteur en verre de 100 mL, muni d'une sonde de mesure de température, d'un barreau aimanté pour assurer une bonne agitation et d'une double enveloppe permettant la circulation d'un liquide de réfrigération, a été purgé d'air et d'humidité, et maintenu à la pression atmosphérique de butène-1. On y a introduit 50,2 mg (0,1 mmole) du complexe Nil₂,[diméthyl-1,3-imidazolylidène-2]₂ et 10 mL d'heptane, puis on a abaissé la température a 10 °C et injecté à l'aide d'une seringue 1,5 mmole de dichloroéthylaluminium dissous dans 2 mL d'heptane. On a mis l'agitation en route et on a observé immédiatement une absorption de butène. Quand le réacteur a été aux trois quarts plein de liquide (au bout d'une demi-heure), on a arrêté l'agitation. A ce moment, on avait introduit au total 24 g de butène. On a produit 4 kg de produits par gramme de Ni pendant une demi-heure. Ces produits sont composés à 90 % en poids de dimères (détermination par chromatographie en phase gazeuse).

## Revendications

1. Composition catalytique résultant de la mise en contact
• d'au moins un complexe du nickel portant au moins un ligand carbène hétérocyclique L ;
• avec au moins un halogénure d'hydrocarbylaluminium ;
• et facultativement avec un solvant organique.

2. Composition catalytique selon la revendication 1 dans laquelle le complexe du nickel répond à la formule générale (NiₐX_{b}Y_{d}L_{c})ⁿ (A)ₙ, dans laquelle
• a, b, c, d et n sont des nombres entiers, avec a égal à 1, 2 ou 3 ; b de 0 à 2 fois a ; d de 0 à 2 fois a ; c de 1 à 4 fois b ; n égal à 0, 1 ou 2 ;
• X et Y, identiques ou différents, représentent chacun un ligand mono- ou multi-chélatant, chargé ou non ;
• L est un mono- ou bi-carbène hétérocyclique ;
• A est un anion faiblement coordinant.

3. Composition catalytique selon la revendication 1 ou 2 **caractérisée en ce que** le ligand L répond à l'une des formules générales (I) et (II) 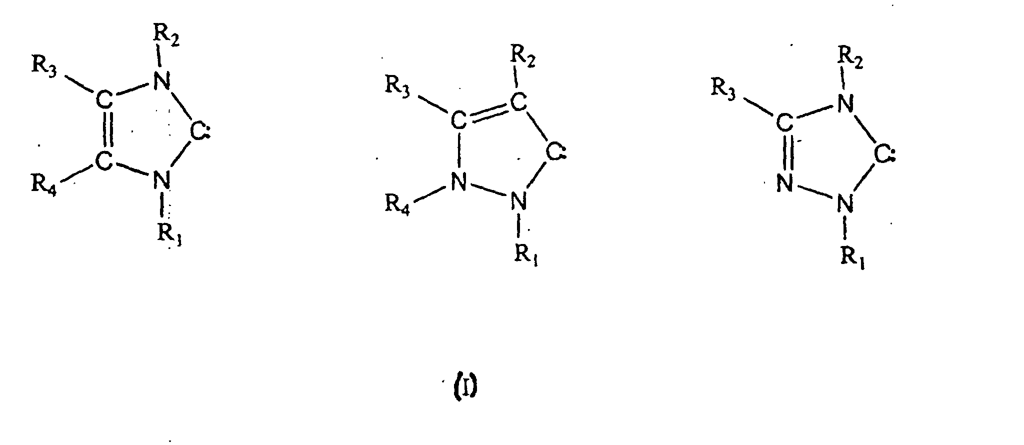 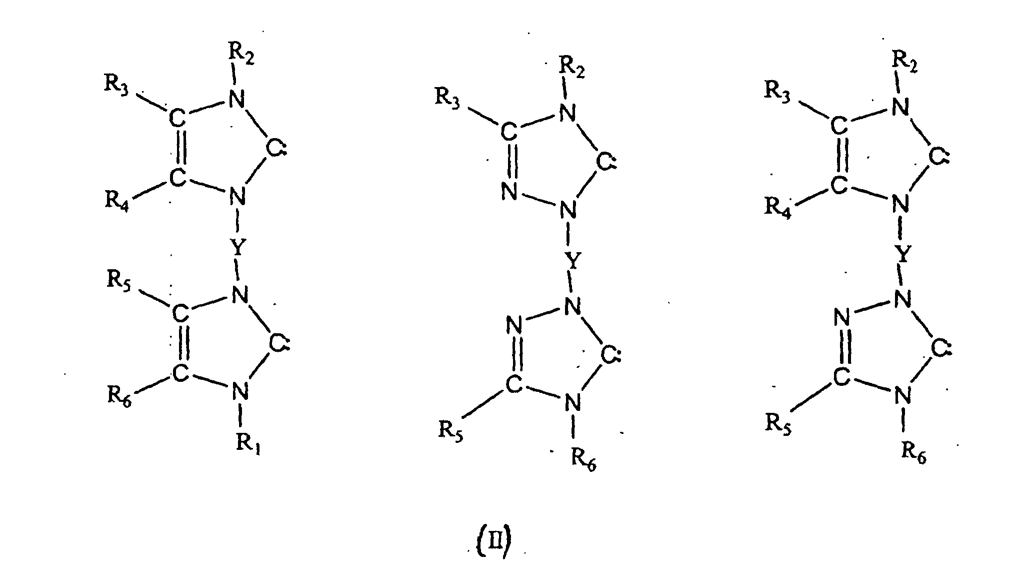 dans lesquelles R₁, R₂, R₃, R₄, R₅ et R₆, identiques ou différents, représentent chacun l'hydrogène, un groupe hydrocarboné, aliphatique, saturé ou insaturé, ou aromatique, comprenant de 1 à 12 atomes de carbone et Y représente un reste bivalent aliphatique de 1 à 4 atomes de carbone.

4. Composition catalytique selon l'une des revendications 1 à 3 **caractérisée en ce que** L est choisi parmi les ligands carbènes répondant aux formules

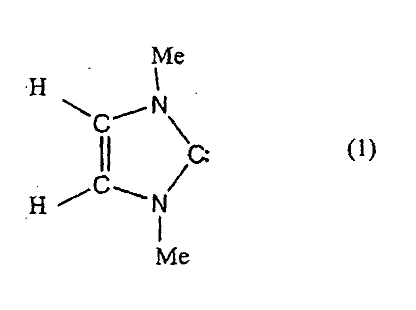

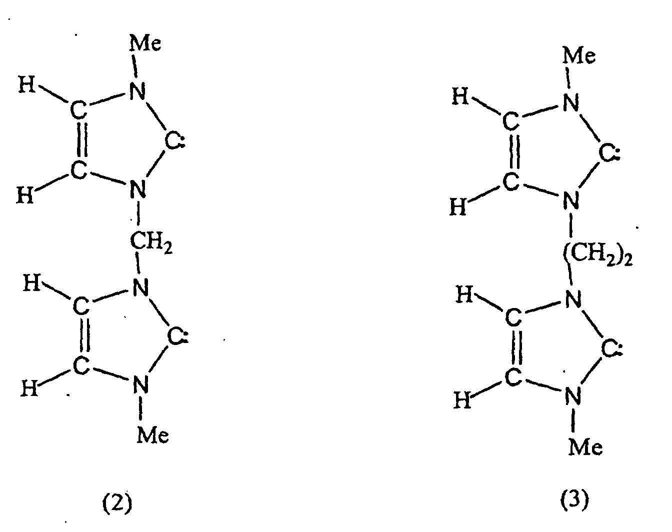

5. Composition catalytique selon l'une des revendications 2 à 4 **caractérisée en ce que** X et Y sont choisis parmi les halogénures, les carboxylates, l'acétylàcétonate, le sulfate, les phénates, les mono- et di-oléfines, les π-aromatiques, les radicaux alkyles ou aryles, les phosphines, les phosphites et le monoxyde de carbone.

6. Composition catalytique selon l'une des revendications 2 à 5 **caractérisée en ce que** A est choisi parmi les anions tétrafluoroborate, hexafluorophosphate, tétraphénylborate et dérivés, tétrachloroaluminates, hexafluoroantimonate, trifluorométhylacétate, trifluorométhylsulfonate et acétate.

7. Composition catalytique selon l'une des revendications 2 à 6 **caractérisée en ce que** le complexe du nickel est choisi parmi
- NiCl₂, [diméthyl-1,3-imidazolylidène-2]₂ ;
- Nil₂,[diméthyl-1,3-imidazolylidène-2]₂ ;
- le chlorure de π-allyl nickel (diméthyl-1,3-imidazolylidène-2) ;
- Nil₂,[diméthyl-1,1'-imidazol-diylidène-2,2'-méthylène-3,3']₂; et
- Nil₂,[diméthyl-1,1'-imidazol-diylidène-2,2'-éthylène-3,3']₂.

8. Composition catalytique selon l'une des revendications 1 à 7 **caractérisée en ce que** le composé organométallique de l'aluminium répond à la formule générale AlRₓX₃₋ₓ, dans laquelle R est un reste hydrocarboné comportant 1 à 12 atomes de carbone, tels que alkyle, linéaire ou ramifié, cycloalkyle aryle ou aralkyle, X étant le chlore ou le brome et x est un nombre de 0 à 3.

9. Composition catalytique selon la revendication 8 **caractérisée en ce que** l'halogénure d'hydrocarbylaluminium est le sesquichlorure d'isobutyl aluminium, le sesquichlorure d'éthylaluminium, le dichloroisobutylaluminium, le dichloroéthylaluminium ou le chlorodiéthylaluminium.

10. Composition catalytique selon les revendications 1 à 9 **caractérisée en ce que** le rapport du composé organique de l'aluminium au complexe du nickel, exprimé par le rapport Al/Ni, est de 2/1 à 50/1.

11. Composition catalytique selon la revendications 1 à 10 **caractérisée en ce que** le solvant utilisé facultativement est un solvant hydrocarboné choisi parmi les alcanes, les hydrocarbures aromatiques, les hydrocarbures halogénés et la ou l'(es) oléfine(s) produite(s) dans la réaction de dimérisation, de codimérisation ou d'oligomérisation.

12. Procédé de dimérisation, de codimérisation ou d'oligomérisation d'au moins une oléfine **caractérisé en ce que** ladite otéfine est mise en contact avec une composition catalytique selon l'une des revendications 1 à 11.

13. Procédé selon la revendication 12 **caractérisé en ce que** la réaction de dimérisation, de codimérisation ou d'oligomérisation d'oléfine(s) est conduite en système fermé, en système semi-ouvert ou en continu, avec un ou plusieurs étages de réaction, sous agitation et à une température de -40 à +80 °C.

14. Procédé selon l'une des revendications 12 et 13 **caractérisé en ce que** l'(les) oléfine(s) est (sont) choisie(s) parmi l'éthylène, le propylène, les n-butènes et les n-pentènes et leurs mélanges, purs ou dilués par au moins un alcane.

15. Procédé selon l'une des revendications 12 à 14 **caractérisé en ce que** l'(les) oléfine(s) est (sont) contenue(s) dans une «coupe» issue d'un procédé de raffinage du pétrole.

## Patentansprüche

1. Katalytische Zusammensetzung, die aus dem Kontaktieren resultiert von
- wenigstens einem Nickelkomplex, der wenigstens einen heterozyklischen Carbenliganden L trägt;
- mit wenigstens einem Halogenid eines Aluminium-Kohlenwasserstoffs;
- und fakultativ mit einem organischen Lösungsmittel.

2. Katalytische Zusammensetzung nach Anspruch 1, in der der Nickelkomplex der allgemeinen Formel (NiₐX_{b}Y_{d}L_{c})ⁿ(A)ₙ entspricht, in welcher
- a, b, c, d und n ganze Zahlen mit a gleich 1, 2 oder 3; b 0- bis 2-mal a; d 0-bis 2-mal a; c 1- bis 4-mal b; n gleich 0,1 oder 2 sind.
- X und Y identisch oder verschieden jedes einen mono oder mehrfach chelatierenden, gegebenenfalls geladenen Liganden darstellen;
- L ein heterozyklisches Mono- oder Bicarben ist;
- A ein schwach koordinierendes Anion ist.

3. Katalytische Zusammensetzung nach einem der Ansprüche 1 oder 2, **dadurch gekennzeichnet, dass** der Ligand L einer der allgemeinen Formeln (I) und (II) entspricht 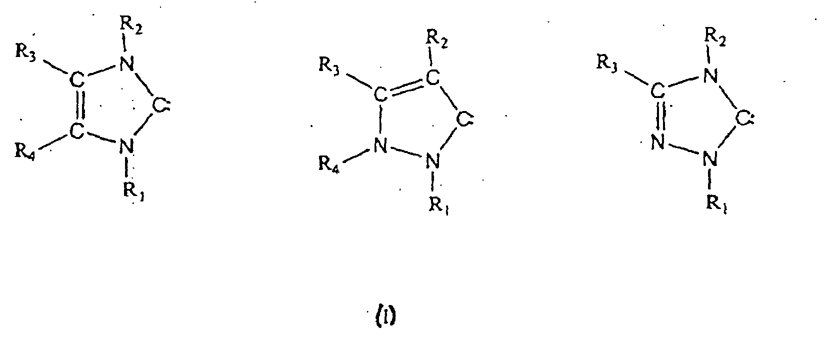 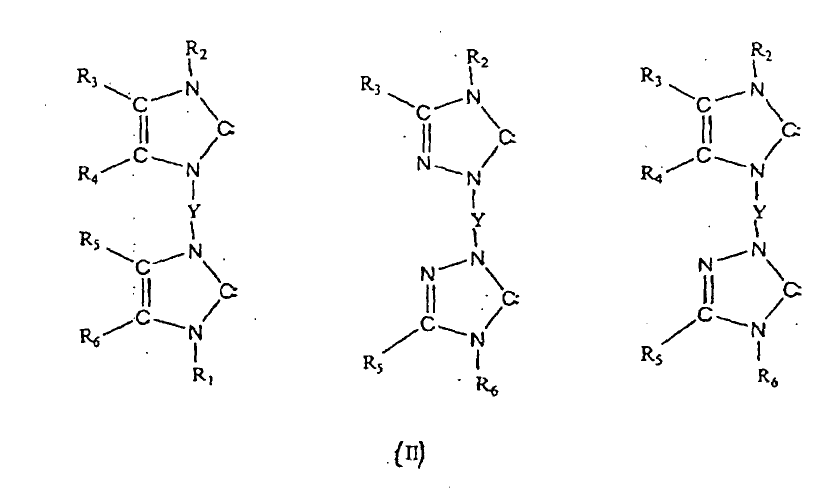 in denen R₁, R₂, R₃ R₄ R₅ und R₆ identisch oder verschieden jedes Wasserstoff, eine Kohlenwasserstoffgruppe, aliphatisch, gesättigt oder ungesättigt oder aromatisch darstellen, die 1 bis 12 Kohlenstoffatome umfasst und Y einen bivalenten aliphatischen Rest mit 1 bis 4 Kohlenstoffatomen darstellt.

4. Katalytische Zusammensetzung nach einem der Ansprüche 1 bis 3, **dadurch gekennzeichnet, dass** L gewählt ist unter den Carbenliganden, die den Formeln entsprechen.

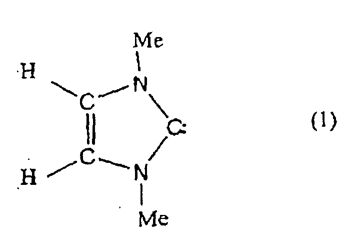

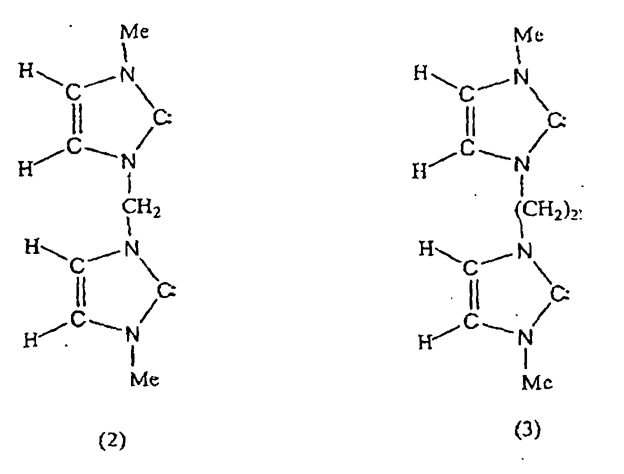

5. Katalytische Zusammensetzung nach einem der Ansprüche 2 bis 4, **dadurch gekennzeichnet, dass** X und Y gewählt sind unter den Halogeniden, den Carboxylaten, Acetylacetonat, Sulfat, Phenaten, den Mono- und Diolefinen, π-Aromaten, Alkyl- oder Arylresten, Phosphinen, Phosphiten und Kohlenmonoxid.

6. Katalytische Zusammensetzung nach einem der Ansprüche 2 bis 5, **dadurch gekennzeichnet**, das A gewählt wird unter den Anionen Tetrafluorborat, Hexaflourphosphat, Tetraphenylborat und Derivaten, Tetrachloraluminaten, Hexaflourantimonat, Triflourmethylacetat, Triflourmethylsulfonat und Acetat.

7. Katalytische Zusammensetzung nach einem der Anspüche 2 bis6, **dadurch gekennzeichnet, dass** der Nickelkomplex gewählt wird aus
- NiCl₂, [1,3-Dimethyl-Imidazol-2-yliden]₂;
- Nil₂, [1,3-Dimethyl-Imidazol-2-yliden]₂;
- π-Allyl-Nickel(1,3-Dimethyl-Imidazol-2-yliden)Chlorid;
- Nil₂, [1,1'-Dimethyl-Imidazol-2-2'-diyliden-3.3'-Methylen]₂; und
- Nil₂, [1,1'-Dimethyl- Imidazol-2-2'-diyliden-3,3'-Ethylen]₂.

8. Katalytische Zusammensetzung nach einem der Ansprüche 1 bis 7, **dadurch gekennzeichnet, dass** die organometallische Aluminiumverbindung der allgemeinen Formel AlRₓX₃₋ₓ entspricht, in der R ein Kohlenwasserstoffrest ist, der 1 bis 12 Kohlenstoffatome umfasst, wie lineares oder verzweigtes Alkyl, Cycloalkyl, Aryl oder Aralkyl, wobei X Chlor oder Brom ist und x eine Zahl von 0 bis 3 ist.

9. Katalytische Zusammensetzung nach Anspruch 8, **dadurch gekennzeichnet, dass** das Halogenid eines Aluminium-Kohlenwasserstoffs, Isobutylaluminium-Sesquichlorid, und Ethylaluminium-Sesquichlorid, Dichlor-Isobutylaluminium, Dichlor-Ethylaluminium oder Chlor-Diethylaluminium ist.

10. Katalytische Zusammensetzung nach den Ansprüchen 1 bis 9, **dadurch gekennzeichnet, dass** das Verhältnis der organischen Aluminiumverbindung zum Nickelkomplex, ausgedrückt durch das Verhältnis Al/Ni 2/1 bis 50/1 ist.

11. Katalytische Zusammensetzung nach den Ansprüchen 1 bis 10, **dadurch gekennzeichnet, dass** das fakultativ verwendete Lösungsmittel ein Kohlenwasserstofflösungsmittel ist, gewählt unter den Alkanen, den aromatischen Kohlenwasserstoffen, den Halogenkohlenwasserstoffen und dem (oder den) in der Reaktion zur Dimerisierung, Codimerisierung oder Oligomerisierung erzeugten Olefin(en).

12. Verfahren zur Dimerisierung, Codimerisierung oder Oligomerisierung von wenigstens einem Olefin, **dadurch gekennzeichnet, dass** das Olefin mit einer katalytischen Zusammensetzung nach einem der Ansprüche 1 bis 11 kontaktiert wird.

13. Verfahren nach Anspruch 12, **dadurch gekennzeichnet, dass** die Reaktion zur Dimerisierung, Codimerisierung oder Oligomerisierung von Olefin(en) im geschlossenen System, halboffenen oder offenen System oder kontinuierlich mir einer oder mehreren Reaktionsstufen unter Rühren und bei einer Temperatur von -40 bis +80°C durchgeführt wird.s

14. Verfahren nach einem der Ansprüche 12 und 13, **dadurch gekennzeichnet, dass** das (die) Olefin(e) gewählt ist (sind) unter Ethylen, Propylen, n-Butenen, n-Pentenen und deren Gemischen rein oder verdünnt durch wenigstens ein Alkan.

15. Verfahren nach einem der Ansprüche 12 bis 14, **dadurch gekennzeichnet, dass** das (die) Olefin(e) enthalten ist (sind) in einer "Fraktion" aus einem Erdölraffinierungsverfahren.

## Claims

1. Catalytic composition that results from bringing into contact
• at least one nickel complex that carries at least one heterocyclic carbene ligand L;
• with at least one hydrocarbylaluminum halide;
• and optionally with an organic solvent.

2. Catalytic composition according to claim 1, in which the nickel complex corresponds to general formula (NiₐX_{b}Y_{d}L_{c})ⁿ (A)ₙ, in which
-- a, b, c, d and n are integers with a equal to 1, 2 or 3; b equal to 0 to 2 x a; d equal to 0 to 2 x a; c equal to 1 to 4 x b; and n equal to 0, 1 or 2;
-- X and Y, identical or different, each represent a mono- or multi-chelating ligand that may or may not be charged;
-- L is a heterocyclic mono- or bi-carbene;
-- A is a sparingly coordinating anion.

3. Catalytic composition according to claim 1 or 2, **characterized in that** ligand L corresponds to one of general formulas (I) and (II) 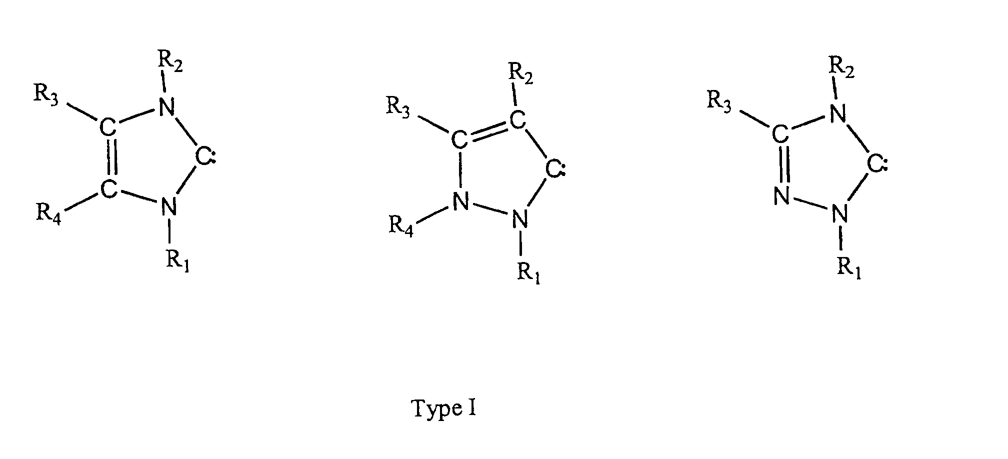 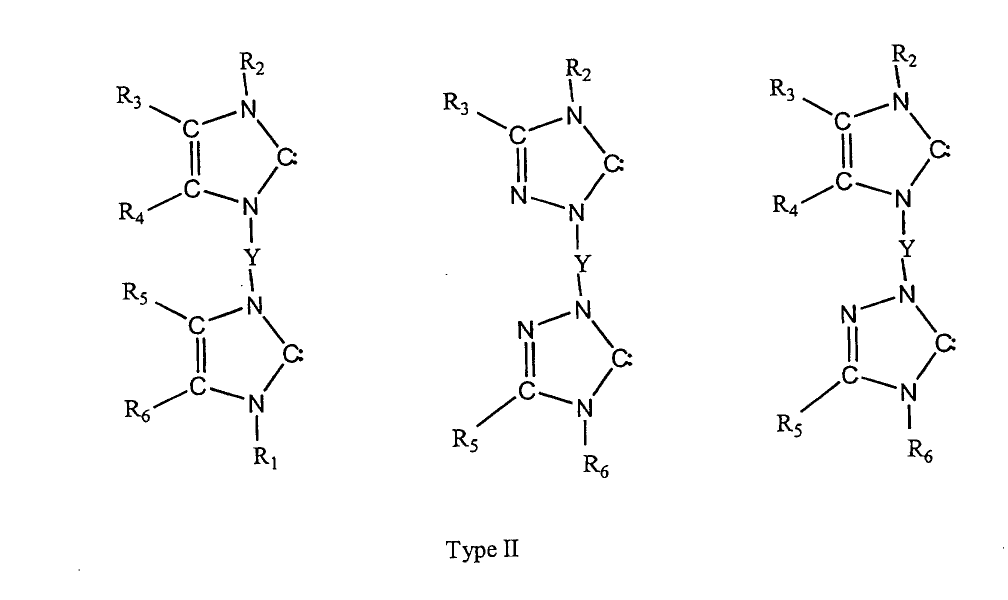 in which R₁, R₂, R₃, R₄, R₅ and R₆, identical or different, each represent hydrogen, a hydrocarbon-containing group, aliphatic group, saturated or unsaturated group, or aromatic group, comprising 1 to 12 carbon atoms, and Y represents an aliphatic bivalent radical with 1 to 4 carbon atoms.

4. Catalytic composition according to one of claims 1 to 3, wherein L is selected from among the carbene ligands that correspond to formulas (1), (2) and (3)

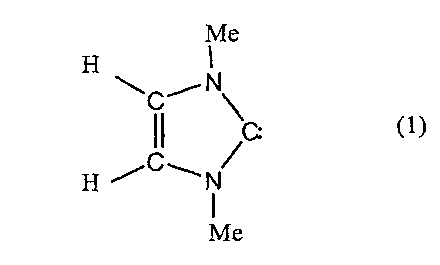

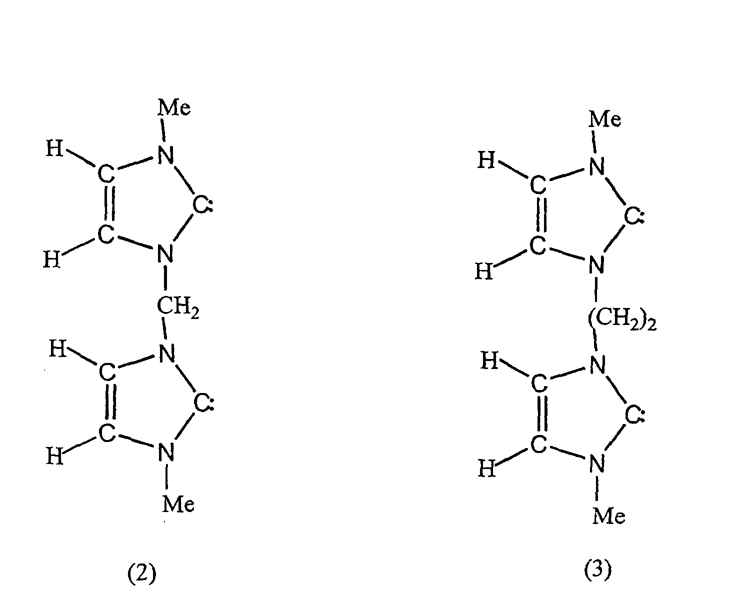

5. Catalytic composition according to one of claims 2 to 4, wherein X and Y are selected from among the halides, carboxylates, acetylacetonate, sulfate, phenates, mono- and di-olefins, π-aromatic compounds, alkyl or aryl radicals, phosphines, phosphites and carbon monoxide.

6. Catalytic composition according to one of claims 2 to 5, wherein A is selected from among the anions of tetrafluoroborate, hexafluorophosphate, tetraphenylborate and derivatives, tetrachloroaluminates, hexafluoroantimonate, trifluoromethylacetate, trifluoromethylsulfonate and acetate.

7. Catalytic composition according to one of claims 2 to 6, wherein the nickel complex is selected from among
-- NiCl₂,[dimethyl-1,3-imidazolylidene-2]₂;
-- Nil₂,[dimethyl-1,3-imidazolylidene-2]₂;
-- π-allyl nickel chloride (dimethyl-1,3-imidazolylidene-2);
-- Nil₂,[dimethyl-1,1'-imidazole-diylidene-2,2'-methylene-3,3']₂; and
-- Nil₂,[dimethyl-1,1'-imidazole-diylidene-2,2'-ethylene-3,3']₂.

8. Catalytic composition according to one of claims 1 to 7, wherein the organometallic aluminum compound corresponds to general formula AlRₓX₃₋ₓ, in which R is a hydrocarbon-containing radical that comprises 1 to 12 carbon atoms, such as alkyl, linear or branched, cycloalkyl, aryl or aralkyl, whereby X is chlorine or bromine and x is a number from 0 to 3.

9. Catalytic composition according to claim 8, wherein the hydrocarbylaluminum halide is isobutylaluminium sesquichloride, ethylaluminum sesquichloride, dichloroisobutylaluminum, dichloroethylaluminum or chlorodiethylaluminum.

10. Catalytic composition according to claims 1 to 9, wherein the ratio of the organic aluminum compound to the nickel complex, expressed by the Al/Ni ratio, is 2/1 to 50/1.

11. Catalytic composition according to claims 1 to 10, wherein the solvent that is optionally used is a hydrocarbon-containing solvent that is selected from among the alkanes, the aromatic hydrocarbons, the halogenated hydrocarbons and the olefin(s) produced in the reaction of dimerization, codimerization or oligomerization.

12. Process of dimerization, codimerization or oligomerization of at least one olefin, wherein said olefin is brought into contact with a catalytic composition according to one of claims 1 to 11.

13. Process according to claim 12, wherein the reaction of dimerization, codimerization or oligomerization of the olefin(s) is conducted in a closed system, semi-open system or continuously, with one or more reaction stages, while being stirred and at a temperature of-40 to +80°C.

14. Process according to one of claims 12 and 13, wherein the olefin(s) is (are) selected from among ethylene, propylene, n-butenes and n-pentenes and mixtures thereof that are pure or diluted by at least one alkane.

15. Process according to one of claims 12 to 14, wherein the olefin(s) is (are) contained in a "fraction" that is obtained from a petroleum refining process.
